(19) Europäisches Patentamt
European Patent Office
Office européen des brevets

(11) Publication number: 0 134 615 B1

(12) # EUROPEAN PATENT SPECIFICATION

(45) Date of publication of patent specification: 04.11.92

(51) Int. Cl.[5]: C07D 279/06, C07F 9/6544, C07C 323/02, C07C 381/00, C07F 9/24

(21) Application number: 84201187.6

(22) Date of filing: 15.08.84

The file contains technical information submitted after the application was filed and not included in this specification

(54) **Process for preparing N-substituted nitromethylene heterocyclic compounds.**

(30) Priority: 26.08.83 GB 8323060

(43) Date of publication of application: 20.03.85 Bulletin 85/12

(45) Publication of the grant of the patent: 04.11.92 Bulletin 92/45

(84) Designated Contracting States: AT BE CH DE FR GB IT LI LU NL SE

(56) References cited:
EP-A- 0 115 323
DE-A- 2 514 402
US-A- 4 006 156
US-A- 4 052 388
US-A- 4 065 560

(73) Proprietor: SHELL INTERNATIONALE RESEARCH MAATSCHAPPIJ B.V.
Carel van Bylandtlaan 30
NL-2596 HR Den Haag(NL)

(72) Inventor: Harris, Martin
54 Northwood Drive
Sittingbourne Kent(GB)

EP 0 134 615 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a process for the preparation of N-acyl nitromethylene thiazines.

Nitromethylene heterocyclic compounds are known to possess an interesting level of insecticidal activity - see for example UK Patent Specification 1,513,951 in which the following compound is particularly exemplified:-

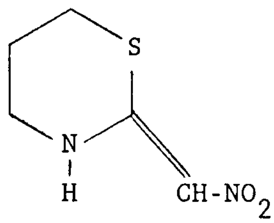

(A)

Hitherto N-substituted derivatives of Compound A and analogues thereof, have been synthesised by derivatising the heterocyclic Compound A or its analogues - see for example UK 1.513.951 page 5 lines 55-61. However attempts to synthesise certain N-acyl derivatives by the direct acylation of Compound A and its analogues failed to yield the desired derivatives. Moreover the synthesis of Compound A involved the use of hazardous intermediates and thus a route to N-acyl derivatives avoiding Compound A was desirable.

EP-A-115 323 discloses a process for producing a heterocyclic compound having a nitromethylene group as the side chain, of the formula:

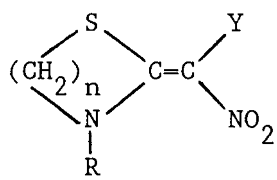

wherein Y represents a hydrogen atom, a halogen atom or a lower alkyl group, R represents a hydrogen atom, an alkyl group, a cycloalkyl group, an aralkyl group, an alkenyl group or an alkynyl group, and n represents an integer of 2, 3 or 4, which comprises allowing a 2,2-dihalonitroethylene compound of the formula:

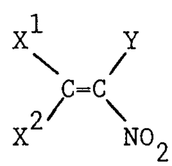

wherein $X^1$ and $X^2$ may be the same or different and represent halogen atoms, and Y is as defined above, to react with a 1-aminoalkanethiol compound or a 1-N-substituted derivative thereof, of the formula:

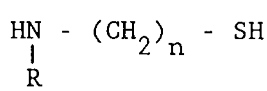

wherein R is as defined above, and n is as defined above, in the presence of a base and in a solvent.

US-A-4,052,388 discloses 3-acetyltetrahydro-2-(nitromethylene)-2H-1,3-thiazine and its use as an insecticide, together with its preparation by reacting 1-nitro-1-(tetrahydro-2H-1,3-thiazin-2-ylidene)-2-pentanone and sodium hydride in monoglyme together with acetyl chloride.

US-A-4006156 discloses 3-alkyl-2-(nitromethylene)thiazolidines having insecticidal activity which are prepared by reacting nitroketene dimethyl mercaptole (ie. 1,1-bis(methylthio)-2-nitroethylene) with a 2-alkylamino-1-mercaptoethane.

US-A-4065560 discloses tetrahydro-2-(nitromethylene)-2H-1,3-thiazines which may be prepared by treating a nitroketene dimethyl mercaptole(ie. 1,1-bis(methylthio)2-nitroethylene) with a 3-amino-1-propanethiol.

DE-A-2514402 discloses 2-nitromethylene imidazolidines and 2-nitromethylene hexahydropyrimidines prepared by the reaction of a diamino alkane with a 1-nitroethylene compound.

The Applicant has found a new process for the manufacture of N-substituted derivatives of Compound A and its analogues which process avoids the need to use the heterocyclic intermediate A. The new process is attractive in that it not only makes available certain known compounds, it also yields novel compounds hitherto inaccessible by other routes. The novel heterocyclic compounds are claimed in copending application EP-A-135 956.

Accordingly the present invention provides a process for preparing an N-acyl nitromethylene thiazine of the general formula:-

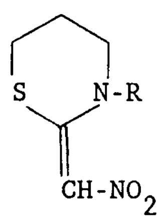

(I)

wherein R represents a formyl group, an alkanoyl group containing 2 to 11 carbon atoms, a fluoroalkanoyl group containing 2 to 11 carbon atoms and 1 to 5 fluorine atoms, a benzoyl group, an optionally brominated phenylsulphonyl group, an alkylaminosulphonyl group bearing two alkyl substituents or a dialkylcarbamoyl group, where each alkyl group contains 1 to 4 carbon atoms, or a group of formula (alkyl-O)$_2$PX- wherein each alkyl group contains 1 to 4 carbon atoms and X is O or S, characterised in that an amide of the following general formula:-

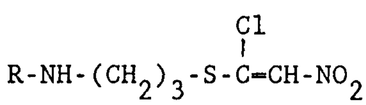

(II)

wherein R has the above meaning, is reacted with a base in the presence of an inert solvent at a temperature between -30 °C and +50 °C, the base being an alkali metal or alkaline earth metal hydride or alkoxide or an alkali metal amide.

Any aliphatic group present in the groups R preferably contains up to 4 carbon atoms.

Suitable bases for use in the process of the invention are sodium or potassium hydride, sodium or potassium alkoxide in which the alkyl group contains 1 to 8 carbon atoms and is preferably branched, e.g. a tertiary alkyl group, and sodium, potassium or lithium amides or dialkylamides wherein each alkyl group contains 1 to 8 carbon atoms, e.g. sodamide and sodium di-isopropylamide.

Any suitable solvent may be used, for example tertiary alcohols e.g. t-butylalcohol, ethers e.g. diethyl ether, tetrahydrofuran and dimethoxyethane, ketones e.g. acetone, nitrogenous solvents e.g. dimethylformamide, hydrocarbons e.g. benzene, xylene and toluene, and sulphur-containing solvents, e.g. dimethyl sulphoxide.

The reaction may proceed at room temperature but in general it can take place at temperatures between -30° and +50 °C, suitably between -20° and +30 °C.

The molar ratio of the reactants is unimportant, e.g. the molar ratio of base to amide can be in the range 1:2 to 2:1 but generally speaking it is preferred to have the basic reactant in excess, i.e. the molar ratio is preferably in the range 1:1 to 2:1.

The N-substituted-S-substituted aminothiol starting materials of general formula II are novel compounds, and form a further aspect of the invention. They may be prepared by a process which comprises reacting an aminothiol of the general formula:-

$SH - (CH_2)_3 - NR$ (III)

wherein R has the meanings hereinbefore specified with a halonitroethylene of the general formula

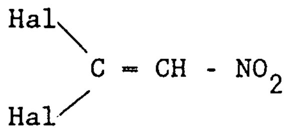 (IV)

wherein Hal is a halogen atom, in the presence of a base which is a hydroxide, carbonate, bicarbonate or acetate of an alkali or alkaline earth metal or of ammonium, and in the presence of an inert solvent. Conveniently the base is an alkali metal hydroxide such as sodium or potassium hydroxide. The inert solvent can for example be any one of the solvents referred to hereinbefore.

It is generally preferred to isolate the amide of general formula II before subjecting it to the action of a base to form the compound I. However isolation of the intermediate II is inessential and the compound I can be made directly by reacting the aminothiol III with the halonitroethylene IV in the presence of base.

The aminothiol compounds of general formula III may be prepared by N-acylation of the corresponding unsubstituted aminothiol.

The process according to the invention is illustrated by the following Examples. Examples 1 to 11 illustrate the synthesis of the novel N-substituted-S-substituted-aminothiols (II), while Examples 12 to 19 illustrate conversion into the compounds of formula I.

Example 1 Preparation of N-acetyl-S-(1-chloro-2-nitroethenyl)-3-aminopropanethiol

To N-acetyl-3-aminopropanethiol (400mg) in methanol (5ml) was added sodium hydroxide (120mg) in methanol (5ml) over 10 minutes. This mixture was then added to 1,1-dichloro-2-nitroethene (950mg) in methanol (5ml) at 0 ° C over a period of 20 minutes. It was stirred for a further 20 minutes and then poured into 2% HCl saturated with NaCl and extracted with methylene chloride. The product was purified over a silica gel column. Yield 400mg; oil.

Example 2 Preparation of N-phenylsulphonyl-S-(1-chloro-2-nitroethenyl)-3-aminopropanethiol

To the hydrochloride salt of 3-amino-propanethiol (5g) was added sodium methoxide (Nalg; methanol 25ml) and the mixture allowed to cool. The solvent was removed. Methylene chloride (60ml) and triethylamine (6g) were added to the product and phenylsulphonyl chloride (7g) in methylene chloride (40ml) added dropwise thereto at 0 ° C over 30 minutes. After 20 minutes at room temperature the product was washed with 2% HCl and triturated with diethyl ether. Yield 6.2g of the disulphide, bis-(N-phenylsulphonyl-3-aminopropyl)-disulphide.

The disulphide (3.1g) was treated with triphenylphosphine (2.5g) in water (2.5g), methanol (50ml) and 10 drops of 10% HCl and refluxed for 11 hours. Most of the solvent was removed and the resulting product was taken up in methylene chloride and washed with water. The product was purified over a silica gel column. Yield of title product 1.6g.

| Analysis: | | | |
|---|---|---|---|
| Calculated | C 46.8; | H 5.6; | N 6.1% |
| Found | C 46.8; | H 5.8; | N 5.9% |

To N-phenylsulphonyl-3-aminopropanethiol (231mg) in methanol (5ml) sodium hydroxide (80mg) in methanol (3ml) was added over 5 minutes and this mixture was then added dropwise to, 1,1-dichloro-2-nitroethene (284mg) in methanol (6ml) at 0 ° C over 20 minutes. The reaction mixture was maintained at 0 ° C for 30 minutes and then poured into 2% HCl saturated with NaCl and extracted with methylene chloride. The product was purified over a silica gel column. Yield 93mg; m.p. 105-106 ° C.

| Analysis | | | |
|---|---|---|---|
| Calculated: | C 39.2; | H 3.9; | N 8.3% |
| Found: | C 39.6; | H 3.8; | N 8.2% |

Example 3 Preparation of N-benzoyl-S-(2-nitro-1-chloroethenyl)-3-aminopropanethiol

To N-benzoyl-3-aminopropanethiol (780mg) in methanol (12ml) at 0°C under nitrogen was added dropwise sodium hydroxide (160mg) in ethanol (6ml). This mixture was then added dropwise at 0°C under nitrogen to 1,1-dichloro-2-nitroethene (1.2g) in methanol (20ml) over 20 minutes. The reaction mixture was poured into 2% HCl saturated with NaCl and extracted with methylene chloride. The product was purified using a silica gel column. Yield 252mg.

| Analysis | | | |
|---|---|---|---|
| Calculated:<br>Found | C 47.9;<br>C 48.3; | H 4.3;<br>H 4.3; | N 9.3%<br>N 9.3% |

Examples 4-11

By methods analogous to those described in Examples 1 to 3, the compounds of Formula I detailed in Table I were prepared.

TABLE I

| Example No. | In the general formula I: R | n | Hal | M.pt °C | Elemental Analysis | C | H | N |
|---|---|---|---|---|---|---|---|---|
| 4 | $CF_3CO$ | 3 | Cl | Oil | Calc. | 28.7 | 2.7 | 9.6 |
| | | | | | Found | 28.5 | 2.9 | 9.4 |
| 5 | $(CH_3)_3C.CO$ | 3 | Cl | | Calc. | 42.8 | 6.1 | 10.0 |
| | | | | | Found | 42.7 | 5.9 | 9.4 |
| 6 | $(C_2H_5O)_2P(S)-$ | 3 | Cl | Oil | Calc. | 31.0 | 5.2 | 7.9 |
| | | | | | Found | 31.0 | 5.2 | 8.0 |
| 7 | $(C_6H_5O)_2P(O)-$ | 3 | Cl | Oil | Calc. | 32.4 | 5.7 | 7.8 |
| | | | | | Found | 32.5 | 5.4 | 8.4 |
| 8 | $(CH_3)_2NCO-$ | 3 | Cl | Oil | Calc. | | | |
| | | | | | Found | not measured | | |
| 9 | 4-bromophenylsulphonyl | 3 | Cl | 96-97 | Calc. | 31.8 | 2.9 | 6.7 |
| | | | | | Found | 31.9 | 2.7 | 6.6 |
| 10 | Dimethylaminosulphonyl | 3 | Cl | Oil | Calc. | 27.7 | 4.6 | 13.8 |
| | | | | | Found | 28.3 | 4.7 | 13.5 |
| 11 | H.CO- | 3 | Cl | | Calc. | 32.7 | 4.7 | 11.6 |
| | | | | | Found | 32.8 | 4.1 | 11.5 |

Example 12 Preparation of N-benzoyl-2-nitromethylene-2H-1,3-thiazine

To N-benzoyl-S-(1-chloro-2-nitroethenyl)-3-aminopropanethiol (233mg) in t-butanol (10ml) under nitrogen was added dropwise potassium t-butoxide (100mg) in t-butanol (3ml) over 5 minutes at room temperature. After 40 minutes the reaction mixture was poured into NaCl/HCl (2%) and extracted with methylene chloride. The product was purified on a silica gel column. Yield 60mg m.p. 90-92° C.

| Analysis | | | |
|---|---|---|---|
| Calculated | C 54.5; | H 4.5; | N 10.6% |
| Found | C 53.8; | H 5.2: | N 10.1% |

Example 13 Preparation of N-acetyl-2-(nitromethylene)-2H-1,3-thiazine

To N-acetyl-S-(1-chloro-2-nitroethenyl)-3-aminopropanethiol (70mg) in t-butanol (3ml) under nitrogen was added potassium t-butoxide (35mg) in t-butanol (1ml) dropwise over 2 minutes. The reaction mixture was stirred at room temperature for 30 minutes and then poured into 2% HCl saturated with NaCl, and extracted with methylene chloride. The product was purified on a silica gel column using methylene chloride/methanol (98.2) as eluant. Yield 8mg. m.p. 91-92.5°C.

Example 14 Preparation of N-phenylsulphonyl-2-nitromethylene-2H-1,3-thiazine

A) To N-phenylsulphonyl-S-(1-chloro-2-nitroethenyl)-3-aminopropanethiol (100mg) in t-butanol (5ml) was added potassium t-butoxide (35mg) in t-butanol (2ml) over 6 minutes. After 20 minutes at room temperature the reaction mixture was poured into 2% HCl saturated with NaCl and then extracted with methylene chloride. The product was purified on a silica gel column. Yield 54mg; m.p. 107°C

| Analysis | | | |
|---|---|---|---|
| Calculated: | C 44.0; | H 4.0; | N 9.2 |
| Found | C 44.0; | H 4.0; | N 9.3 |

B) To N-phenylsulphonyl-3-aminopropanethiol (231mg) in t-butanol (5ml) was added potassium t-butoxide (224mg) dropwise over 10 minutes under nitrogen. This mixture was then added dropwise to 1,1-dichloro-2-nitroethene (300mg) under nitrogen over 20 minutes. After 20 minutes at room temperature the reaction mixture was poured into 2% HCl saturated with NaCl, and extracted with methylene chloride. The product was purified on a silica gel column using methylene chloride as eluant. Yield 31mg; m.p. 106-107°C.

| Analysis | | | |
|---|---|---|---|
| Calculated: | C 44.0; | H 4.0; | N 9.3% |
| Found | C 43.9; | H 4.0; | N 9.2% |

C) Butyllithium (0.33 ml) was added under nitrogen to diisopropylamine (50 mg) in tetrahydrofuran (6ml) at -78°C. The resulting mixture was added dropwise to a solution of N-phenylsulphonyl-S-(1-chloro-2-nitroethenyl)-3-aminopropanethiol (146 mg) in tetrahydrofuran (6ml) at -78°C. The mixture was allowed to warm to room temperature over 20 minutes, and was then worked up as described in Example 14A. 86mg of the desired product was obtained, m.pt. 106-107°C.

Example 15 Preparation of N-(diethoxythiophosphorus)-2-(nitromethylene)-2H-1,3-thiazine

To N-(diethoxythiophosphorus)-S-(1-chloro-2-nitroethenyl)3-aminopropanethiol (180mg) in tertiary-butanol (10ml) was added potassium t-butoxide (65mg) in t-butanol (4ml) dropwise over 5 minutes. After 30 minutes at room temperature the reaction mixture was poured into 2% HCl saturated with NaCl and extracted with ethylene chloride. The product was purified on a silica gel column using ethylene chloride as eluant. Yield 76mg; oil.

Example 16 Preparation of N-(p-bromophenylsulphonyl)-2-nitromethylene-2H-1,3-thiazine

To N-(p-bromophenylsulphonyl)-S-(1-chloro-2-nitroethenyl)-3-aminopropanethiol (500mg) in tetrahydrofuran (40ml) at 0°C under nitrogen was add potassium t-butoxide (200mg) as solid over 5 minutes. The mixture was stirred at room temperature for 15 minutes and then worked up as in Example 14A. Yield 300mg; m.pt. 96-97°C.

| Analysis | | | |
|---|---|---|---|
| Calculated | C 31.8 | H 2.9 | N 6.7 |
| Found | C 31.9 | H 2.7 | N 6.6 |

Examples 17 to 19

The process according to the invention was also carried out in order to prepare further compounds of the general formula I. Details are given in Table II. In all cases, Hal in formula II was chlorine, and n was 3.

TABLE II

| Example No. | R in formulae I and II | M.Pt. °C | Analysis of Product Elemental Analysis | C | H | N |
|---|---|---|---|---|---|---|
| 17 | $(CH_3)_2NCO-$ | 104-106 | Calc. | 41.6 | 5.6 | 18.2 |
| | | | Found | 41.6 | 5.4 | 17.0 |
| 18 | $(CH_3)_2NSO_2-$ | 92-93 | Calc. | 31.5 | 4.9 | 15.7 |
| | | | Found | 31.7 | 4.9 | 15.6 |
| 19 | HCO- | 132 | Not measured | | | |

## Claims

**Claims for the following Contracting States : BE, CH, DE, FR, GB, IT, LI, LU, NL, SE**

**1.** A process for preparing an N-acyl nitromethylene thiazine of the general formula:-

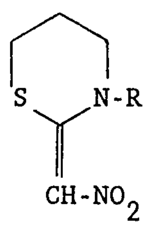

wherein R represents a formyl group, an alkanoyl group containing 2 to 11 carbon atoms, a fluoroalkanoyl group containing 2 to 11 carbon atoms and 1 to 5 fluorine atoms, a benzoyl group, an optionally brominated phenysulphonyl group, an alkylaminosulphonyl group bearing two alkyl substituents or a dialkylcarbamoyl group where each alkyl group contains 1 to 4 carbon atoms, or a group of formula (alkyl-O)$_2$PX- wherein each alkyl group contains 1 to 4 carbon atoms and X is O or S, characterised in that an amide of the following general formula:-

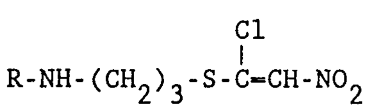 (II)

wherein R has the above meaning, is reacted with a base in the presence of an inert solvent at a temperature between -30 °C and +50 °C, the base being an alkali metal or alkaline earth metal hydride or alkoxide or an alkali metal amide.

**2.** A process according to claim 1 characterised in that the base is sodium or potassium hydride, sodium or potassium alkoxide in which the alkyl group contains 1 to 8 carbon atoms, or sodium, potassium or lithium amide or dialkylamide in which each alkyl group contains 1 to 8 carbon atoms.

**3.** A process according to claim 1 or 2 characterised in that the amide of formula II has been prepared by a process which comprise reacting an aminothiol of the general formula:-

$SH - (CH_2)_3 - NR$ (III)

wherein R has the meaning given in claim 1 with 1-nitro-2,2-dichloroethylene in the presence of a base which is a hydroxide, carbonate, bicarbonate or acetate of an alkali or alkaline earth metal or of ammonium and in the presence of an inert solvent.

**4.** A compound of the general formula

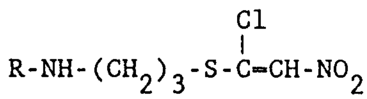

(II)

in which R has the meaning given in claim 1.

**Claims for the following Contracting State : AT**

**1.** A process for preparing an N-acyl nitromethylene thiazine of the general formula:-

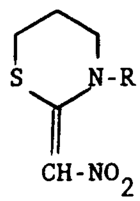

wherein R represents a formyl group, an alkanoyl group containing 2 to 11 carbon atoms, a fluoroalkanoyl group containing 2 to 11 carbon atoms and 1 to 5 fluorine atoms, a benzoyl group, an optionally brominated phenysulphonyl group, an alkylaminosulphonyl group bearing two alkyl substituents or a dialkylcarbamoyl group where each alkyl group contains 1 to 4 carbon atoms, or a group of formula $(alkyl\text{-}O)_2PX$- wherein each alkyl group contains 1 to 4 carbon atoms and X is O or S, characterised in that an amide of the following general formula:-

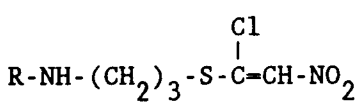

(II)

wherein R has the above meaning, is reacted with a base in the presence of an inert solvent at a temperature between -30 ° C and +50 ° C, the base being an alkali metal or alkaline earth metal hydride or alkoxide or an alkali metal amide.

**2.** A process according to claim 1 characterised in that the base is sodium or potassium hydride, sodium or potassium alkoxide in which the alkyl group contains 1 to 8 carbon atoms, or sodium, potassium or lithium amide or dialkylamide in which each alkyl group contains 1 to 8 carbon atoms.

**3.** A process according to claim 1 or 2 characterised in that the amide of formula II has been prepared by a process which comprise reacting an aminothiol of the general formula:-

$SH - (CH_2)_3 - NR$ (III)

wherein R has the meaning given in claim 1 with 1-nitro-2,2-dichloroethylene in the presence of a base which is a hydroxide, carbonate, bicarbonate or acetate of an alkali or alkaline earth metal or of ammonium and in the presence of an inert solvent.

## Patentansprüche

### Patentansprüche für folgende Vertragsstaaten : BE, CH, DE, FR, GB, IT, LI, LU, NL, SE

**1.** Verfahren zur Herstellung eines N-Acyl-nitromethylenthiazins der allgemeinen Formel:

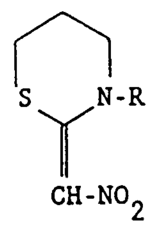

,

worin R eine Formylgruppe, eine Alkanolygruppe mit 2 bis 11 Kohlenstoffatomen, eine Fluoralkanoylgruppe mit 2 bis 11 Kohlenstoffatomen und 1 bis 5 Fluoratomen, eine Benzoylgruppe, eine gegebenenfalls bromierte Phenylsulfonylgruppe, eine Alkylaminosulfonylgruppe, die 2 Alkylsubstituenten oder eine Dialkylcarbamoylgruppe trägt, worin jede Alkylgruppe 1 bis 4 Kohlenstoffatome aufweist, oder eine Gruppe der Formel $(Alkyl\text{-}O)_2PX$-darstellt, worin jede Alkylgruppe 1 bis 4 Koblenstoffstome enthält und X für O oder S steht, dadurch gekennzeichnet, daß ein Amid der nachstehenden allgemeinen Formel

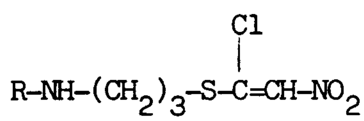

(II),

worin R die obige Bedeutung hat, mit einer Base in Anwesenheit eines inerten Lösungsmittels bei einer Temperatur zwischen -30 °C und +50 °C umgesetzt wird, wobei die Base ein Alkalimetall- oder Erdalkalimetallhydrid oder -alkoxid oder ein Alkalimetallamid ist.

**2.** Verfahren nach Anspruch 1, dadurch gekennzeichnet, daß die Base Natrium- oder Kaliumhydrid, Natrium- oder Kaliumalkoxid, worin die Alkylgruppe 1 bis 8 Kohlenstoffatome enthält, oder Natrium-, Kalium- oder Lithiumamid oder -dialkylamid ist, worin jede Alkylgruppe 1 bis 8 Kohlenstoffatome enthält.

**3.** Verfahren nach Anspruch 1 oder 2, dadurch gekennzeichnet, daß das Amid der Formel II nach einem Verfahren hergestellt worden ist, welches ein Umsetzen eines Aminothiols der allgemeinen Formel:

$SH - (CH_2)_3 - NR$ (III),

worin R die in Anspruch 1 angegebene Bedeutung hat, mit 1-Nitro-2,2-dichlorethylen in Anwesenheit einer Base, die ein Hydroxid, Carbonat, Bicarbonat oder Azetat eines Alkali- oder Erdalkalimetalles oder von Ammonium ist, und in Anwesenheit eines inerten Lösungsmittels umfaßt.

**4.** Eine Verbindung der allgemeinen Formel

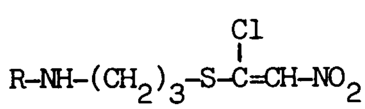

(II),

worin R die in Anspruch 1 angegebene Bedeutung aufweist.

### Patentansprüche für folgenden Vertragsstaat : AT

**1.** Verfahren zur Herstellung eines N-Acyl-nitromethylenthiazins der allgemeinen Formel:

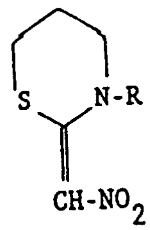

,

worin R eine Formylgruppe, eine Alkanoylgruppe mit 2 bis 11 Kohlenstoffatomen, eine Fluoralkanoylgruppe mit 2 bis 11 Kohlenstoffatomen und 1 bis 5 Fluoratomen, eine Benzoylgruppe, eine gegebenenfalls bromierte Phenylsulfonylgruppe, eine Alkylaminosulfonylgruppe, die 2 Alkylsubstituenten oder eine Dialkylcarbamoylgruppe trägt, worin jede Alkylgruppe 1 bis 4 Kohlenstoffatome aufweist, oder eine Gruppe der Formel (Alkyl-O)$_2$PX-darstellt, worin jede Alkylgruppe 1 bis 4 Kohlenstoffatome enthält und X für O oder S steht, dadurch gekennzeichnet, daß ein Amid der nachstehenden allgemeinen Formel

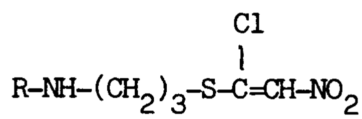

(II),

worin R die obige Bedeutung hat, mit einer Base in Anwesenheit eines inerten Lösungsmittels bei einer Temperatur zwischen -30 ° C und +50 ° C umgesetzt wird, wobei die Base ein Alkalimetall- oder Erdalkalimetallhydrid oder -alkoxid oder ein Alkalimetallamid ist.

2. Verfahren nach Anspruch 1, dadurch gekennzeichnet, daß die Base Natrium- oder Kaliumhydrid, Natrium- oder Kaliumalkoxid, worin die Alkylgruppe 1 bis 8 Kohlenstoffatome enthält, oder Natrium-, Kalium- oder Lithiumamid oder -dialkylamid ist, worin jede Alkylgruppe 1 bis 8 Kohlenstoffatome enthält.

3. Verfahren nach Anspruch 1 oder 2, dadurch gekennzeichnet, daß das Amid der Formel II nach einem Verfahren hergestellt worden ist, welches ein Umsetzen eines Aminothiols der allgemeinen Formel:

$SH - (CH_2)_3 - NR$ (III),

worin R die in Anspruch 1 angegebene Bedeutung hat, mit 1-Nitro-2,2-dichlorethylen in Anwesenheit einer Base, die ein Hydroxid, Carbonat, Bicarbonat oder Azetat eines Alkali- oder Erdalkalimetalles oder von Ammonium ist, und in Anwesenheit eines inerten Lösungsmittels umfaßt.

**Revendications**

**Revendications pour les Etats contractants suivants : BE, CH, DE, FR, GB, IT, LI, LU, NL, SE**

1. Procédé de préparation d'une N-acylnitrométhylènethiazine de formule générale :

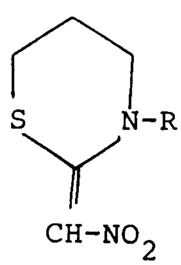

dans laquelle R représente un groupe formyle, un groupe alcanoyle contenant 2 à 11 atomes de carbone, un groupe fluoroalcanoyle contenant de 2 à 11 atomes de carbone et 1 à 5 atomes de fluor, un groupe benzoyle, un groupe phénylsulfonyle éventuellement bromé, un groupe alkylaminosulfonyle

portant deux substituants alkyles ou un groupe dialkylcarbamoyle, dans lequel chaque groupe alkyle contient de 1 à 4 atomes de carbone, ou un groupe de formule $(alkyl\text{-}O)_2PX\text{-}$ dans lequel chaque groupe alkyle contient de 1 à 4 atomes de carbone et X est O ou S, caractérisé en ce qu'on fait réagir un amide de la formule générale suivante :

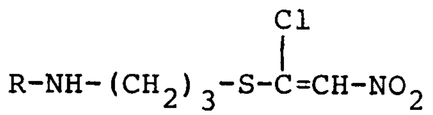

(II)

dans laquelle R a la signification ci-dessus, avec une base en présence d'un solvant inerte à une température comprise entre -30 °C et +50 °C, la base étant un hydrure ou un alcoxyde de métal alcalin ou de métal alcalinoterreux, ou un amide de métal alcalin.

**2.** Procédé selon la revendication 1, caractérisé en ce que la base est de l'hydrure de sodium ou de potassium, un alcoxyde de sodium ou de potassium dans lequel le groupe alkyle contient 1 à 8 atomes de carbone ou un amide ou dialkylamide de sodium, potassium ou lithium, dans lequel chaque groupe alkyle contient 1 à 8 atomes de carbone.

**3.** Procédé selon la revendication 1 ou 2, caractérisé en ce que l'amide de formule II a été préparé par un procédé qui comprend de faire réagir un aminothiol de formule générale :

$SH - (CH_2)_3 - NR$ (III)

dans laquelle R a la signification donnée à la revendication 1 avec du 1-nitro-2,2-dichloroéthylène en présence d'une base qui est un hydroxyde, carbonate, bicarbonate ou acétate d'un métal alcalin ou alcalino-terreux ou de l'ammonium et en présence d'un solvant inerte.

**4.** Composé de formule générale

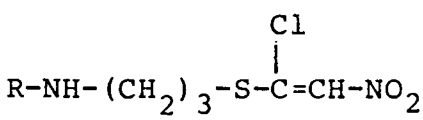

(II)

dans laquelle R a la signification donnée à la revendication 1.

**Revendications pour l'Etat contractant suivant : AT**

**1.** Procédé de préparation d'une N-acylnitrométhylènethiazine de formule générale :

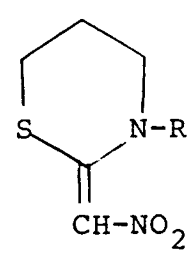

dans laquelle R représente un groupe formyle, un groupe alcanoyle contenant 2 à 11 atomes de carbone, un groupe fluoroalcanoyle contenant de 2 à 11 atomes de carbone et 1 à 5 atomes de fluor, un groupe benzoyle, un groupe phénylsulfonyle éventuellement bromé, un groupe alkylaminosulfonyle portant deux substituants alkyles ou un groupe dialkylcarbamoyle, dans lequel chaque groupe alkyle contient de 1 à 4 atomes de carbone, ou un groupe de formule $(alkyl\text{-}O)_2PX\text{-}$ dans lequel chaque

groupe alkyle contient de 1 à 4 atomes de carbone et X est O ou S, caractérisé en ce qu'on fait réagir un amide de la formule générale suivante :

$$R\text{-}NH\text{-}(CH_2)_3\text{-}S\text{-}\overset{\overset{Cl}{|}}{C}{=}CH\text{-}NO_2 \qquad (II)$$

dans laquelle R a la signification ci-dessus, avec une base en présence d'un solvant inerte à une température comprise entre -30 °C et +50 °C, la base étant un hydrure ou un alcoxyde de métal alcalin ou de métal alcalinoterreux, ou un amide de métal alcalin.

**2.** Procédé selon la revendication 1, caractérisé en ce que la base est de l'hydrure de sodium ou de potassium, un alcoxyde de sodium ou de potassium dans lequel le groupe alkyle contient 1 à 8 atomes de carbone ou un amide ou dialkylamide de sodium, potassium ou lithium, dans lequel chaque groupe alkyle contient 1 à 8 atomes de carbone.

**3.** Procédé selon la revendication 1 ou 2, caractérisé en ce que l'amide de formule II a été préparé par un procédé qui comprend de faire réagir un aminothiol de formule générale :

$SH - (CH_2)_3 - NR$ (III)

dans laquelle R a la signification donnée à la revendication 1 avec du 1-nitro-2,2-dichloroéthylène en présence d'une base qui est un hydroxyde, carbonate, bicarbonate ou acétate d'un métal alcalin ou alcalinoterreux ou de l'ammonium et en présence d'un solvant inerte.